# EUROPEAN PATENT SPECIFICATION

(11) **EP 2 242 497 B1**
(45) Date of publication and mention of the grant of the patent: **04.09.2013**
(21) Application number: 08831804.3
(22) Date of filing: 19.09.2008
(51) Int. Cl.: A61K 36/898, A61P 3/10, A61K 36/00, A61K 35/64, A61K 31/11, A61K 31/355, A61K 31/715, A61K 33/02

(54) **DIABETES PROPHYLAXIS AND TREATMENT REMEDY AND A METHOD OF GETTING IT**
MITTEL ZUR PROPHYLAXE UND BEHANDLUNG VON DIABETES UND DESSEN HERSTELLUNGSMETHODE
REMÈDE POUR LA PRÉVENTION ET LE TRAITEMENT DU DIABÈTE ET PROCÉDÉ D'OBTENTION

(30) Priority: 21.09.2007 BG 10996207
(43) Date of publication of application: 27.10.2010
(73) Proprietor: Zdrave-Tera Ltd, 5500 Lovech (BG)
(72) Inventor: TSACHEVA, Valentina, 5700 Teteven (BG); VASILKOVSKI, Docho, 8000 Burgas (BG); VALEV, Ivan, 5500 Lovech (BG)
(74) Representative: Pakidanska, Ivanka Slavcheva
(86) International application number: PCT/BG2008/000016
(87) International publication number: WO 2009/036529

(56) References cited:
- WO-A-02/30467
- US-A1- 2006 257 502
- DATABASE WPI Week 200578 Thomson Scientific, London, GB; AN 2005-768371 XP002509938 & WO 2005/058069 A (ENGELS E V) 30 June 2005 (2005-06-30)

## Description

### FIELD OF THE INVENTION

The invention concerns a diabetes prophylaxis and treatment remedy and a method of getting it, hereafter referred as "Remedy".

### PRIOR ART

The etiology of diabetes has not been completely clarified yet. The pathological physiology of this disease is defined by the low levels of the endocrine hormone insulin, which regulates the blood sugar levels in the body. Depending on the insulin level, the disease is either non-insulin-dependent or insulin-dependent diabetes.

There are different remedies intended for treatment of both types of diabetes, enumerated below. [1] There is a non-insulin-dependent diabetes treatment with sulfonylurea, stimulating the insulin secretion by increasing the glucose sensibility. A disadvantage of these remedies is their interaction with attendant treatment medications which either increase the risk of causing hypoglycemia (such as silicon derivatives, sulphanamides, beta-blockers, chloramphenicole, alcohol, etc.) or decrease it (such as tiasid diuretics, furozemid, diazoxid, glucose-corticosteroids, glucagon, adrenal antagonists, etc.). This makes the treatment of attendant diseases of diabetics more difficult.

There is also a non-insulin-dependent diabetes treatment with biguanides, which increase the insulin effect by increasing the glucose absorption in the muscle tissues, increasing the permeability of the cell membrane to glucose, decreasing the neoglicogenesis and decreasing the intestine resorption of glucose. A disadvantage of these medications is that they are very toxic and as a result most of them have already fell into disuse.

There is another non-insulin-dependent diabetes treatment with alpha-glucose inhibitors, which delay the hexoses resorption and lead to decreasing the postprandial hyperglycemia. A common disadvantage of the treatment with these medications is the fact that the sick is to strictly see to taking them regularly and monitoring the blood sugar level in order to regulate the needed dose as well as to stay on a strict diet.

As for the insulin-dependent diabetes, there is a treatment with substituting insulin therapy, which includes quick-action and long-action insulin, insulin mixtures and ultra-quick-action insulin analogues. A disadvantage of these medications is that they are applied every day or several times a day orally or percutaneously (the common ones) and every 36 hours (the depot-insulin ones) for life. This leads to unpleasant sensations connected with the injections, a regular measuring of the blood sugar levels in order to regulate the dose needed, necessity of always being on a strict diet, restriction of the natural activity to a certain extent and, as a whole, lowering the quality of the sick's life.

Honey has been known for many years to have a favorable influence on bettering the general condition of the organism. In addition, as well as for prophylaxis and treatment of a number of diseases, such as psychic disorders, infectious diseases, bone-joint and tendon tissues diseases, etc. Despite the common statement that honey is contra-indicated for diabetics, some researches on how honey influences these diseases have shown that applied in small doses honey has a positive effect on lowering the blood sugar levels. [2], [3].

The disadvantage of this treatment is that despite the some positive effect, it can not completely and long-lastingly bring the blood sugar level to normal.

The common disadvantage of all existing methods of diabetes treatment is that the organism itself does not succeed in producing the necessary quantity of insulin and the medications used can not physiologically answer the physiological requirements in case of physical and emotional burdening (stress) as well as in case of interaction with other medications, as a result of which the blood sugar level can not always be kept in acceptable limits and this leads to worsening of the physical health and the neuropsychic equilibrium.

### TECHNICAL FIELD OF THE INVENTION

The objective of the invention is to conceive a remedy for prophylaxis of those who are healthy or in danger of getting diabetes as well as for complete and long-lasting therapy of diabetes, a remedy which is not toxic, has no side effects, is easy to take, does not interact negatively with food-stuffs and medications, does not cause negative emotions, is easy to produce and has low prime cost. Another objective of the invention is to create a method for getting of the above remedy.

This objective is fulfilled by inventing a remedy for use in the diabetes treatment, bettering the neuropsychic equilibrium and the general condition of the organism as a whole.

The active substance in the Remedy works in a completely different way compared to the way the already known diabetes treatment remedies do. While those are aimed at overcoming the negative consequences of the decreased production or complete lack of insulin (delay of monosaccharide resorption, glucose absorption by the muscle tissues, insulin substitutes introducing), the newly invented Remedy attacks the cause for the decreased insulin production.

Based on Oparin's theory of the origin of life and Darwin's theory of the origin of species, a theory is supported that the microorganisms which have originated and followed their independent way of evolution are known from the respective sciences (bacteriology, mycology, virology, hlamidology), and those who have fallen into symbiotic relations among themselves and have been stimulated by the antagonistic and parasitic behavior of others, in the beginning - just microorganisms and after that - macro organisms, have attained their perfection in that symbiotic complex, recognized to be the height of the evolution development in the terrestrial world, called human body. This compound symbiotic complex has not been completely investigated yet. The role of the different components, their relations and functions and their significance for the right functioning of the whole complex has not been fully clarified.

Having in mind the above-mentioned base theories and analyzing the different components and relations in the organism, which is a compound symbiotic complex in which the microorganisms with their chemical and biochemical metabolism are the living bond between the inanimate nature and the macro organisms, the authors reach the conclusion that the main reason for a great number of human and animal diseases is the disturbed harmony among the populations of some microorganisms in the organism. In particular, the prime cause of diabetes is the mass infection of wild and domestic animals and of humans with the micro parasite horse liver fluke, whose harmful influence over animals and humans has been underestimated by the science.

As a result of the metabolism of the micro parasite horse liver fluke metabolic products are obtained, among which naphthalene chloride and sodium nitrate, which oppress the growth of a specific microorganism in the pancreas, whose waste product is the insulin. The graveness of the disease is an equivalent of intoxication affecting not only the production of insulin but also the hormonal, nerve and circulatory systems. The metabolic product of the micro parasite horse liver fluke naphthalene chloride is not thrown out of the organism, but is piled up in the adipose depots of the body, thus keeping to harm proportionally the overweight.

Furthermore, in a not completely explained way, similar to the principle of the vaccines, one of the nuclear chromosomes (of RNA) is engaged in the production of the specific toxin of the micro parasite horse liver fluke - naphthalene chloride and increases repeatedly its quantity in the organism. The other metabolic product - sodium nitrate stimulates the fermentation of relatively low level sugar in the diabetic's organism to methyl alcohol, which completes the clinic of diabetes. This causes a serious disorder of all systems of the body and a complete harm of the organism, becoming the base disease for plenty of other diseases.

The curing effect of the Remedy, according to the invention, is due to a bacterial product - lithium nitrate (LiNo₃) produced through fermentation processes from its components. The lithium nitrate oppresses the growth of the micro parasite horse liver fluke and neutralizes part of its metabolic products, such as naphthalene chloride and sodium nitrate (NaN0₃). What's more, the lithium nitrate stops the cell production of the manipulated nuclear chromosome of the toxin naphthalene chloride and betters the living ambient for the growth of a specific microorganism in the beta-cells of the pancreas, whose waste product is insulin. Due to the favorable living ambient, the specific microorganism in the pancreas self-reproduces and quickly reaches the crucial level of maintaining its population in the symbiotic complex on a primary disease stage. Once restored, the symbiotic relations within the microorganism keep it normal and there is no need to use the Remedy, according to the invention the in future in order to keep its favorable living ambient.

For the insulin-dependent diabetes, where the population of the specific microorganism in the pancreas is too badly affected, the time necessary for the treatment with the Remedy is longer and depends on the vitality of the population of this specific microorganism in the beta-cells, on the nerve system intoxication level and the circulatory system harm level. In the beginning the Remedy is taken in smaller doses, which gradually increase until reaching the normal doses for non-insulin-dependent diabetes.

### COMPOSITION OF THE REMEDY

The Remedy is a fermented product of Bee honey (from 68.07 to 92.74 units), Ammonium chloride (from 0.42 to 2.20 units), Pectin (from 1.86 to 16.94 units), Vanillin (from2,71 to 12.71 units) and Vitamin E (from 0,42 to 1.69 units), the end product containing Lithium nitrate from 0,022 to 0,042 units of the active substance.

### METHOD OF PRODUCTION

According to the invention the method of Remedy production includes the following steps: Ammonium chloride (from 0.42 to 2.20 units) is mixed with Pectin (from 1.86 to 16.94 units) and Vanillin (from 2.71 to 12.71 units) and is stirred well. Then Bee honey (from 68.07 to 92.74 units) is added and the mixture is stirred until it becomes homogeneous. After that Vitamin E (from 0.42 to 1.69 units) is added, stirred well and left in an uncovered container at room temperature until it becomes completely fermented (usually for about 10 days).

The received Remedy is used for prophylaxis and treatment of diabetes (insulin-dependent and non-insulin-dependent) by restoration of the physiological level of the insulin and the level of the blood sugar, as well as for bettering the psychological equilibrium and the general status of the organism.

The treatment of the above-mentioned diseases is performed by passing two or more courses of treatment, each of them lasting for 8 - 20 days. During the course of treatment a certain quantity of the Remedy is taken with water instead of breakfast. Between two courses of treatment with the Remedy there is a 20 to 40-day rest.

For the treatment of non-insulin-dependent diabetes one needs either two or three courses of treatment. The dose per day depends on the level of the insulin insufficiency and varies from 95 to 100 g.

For the treatment of insulin-dependent diabetes it is necessary to pass two or three courses of treatment with 20 to 30 g of the Remedy a day, followed by two or three courses of treatment with 40 to 60 g of the remedy a day, followed by other two or three courses of treatment with 95 to 100 g of the remedy a day.

Simultaneously with the treatment of diabetes and its prophylaxis, the neuropsychic equilibrium and the general status of the organism are also improved.

### DESCRIPTION OF PREFERRED EMBODIMENTS

The invention is illustrated (but not limited to) with the following preferred embodiments of compositions of the Remedy, methods of getting them and examples of their application for treatment of some diseases.

### Preferred embodiment of composition No1 and method of getting it (the most preferable)

A Fermented product containing 84.77 units Bee honey, 1.69 units Ammonium chloride, 4.23 units Pectin, 8.47 units Vanillin and 0.84 units Vitamin E.

The most preferred embodiment 1 is received in the following way:
1) 1.69 units Ammonium chloride, 4.23 units Pectin and 8.47 units Vanillin are mixed and well stirred;
2) 84.77 units Bee honey are added and the mixture is stirred until it becomes homogeneous;
3) 0.84 units Vitamin E is added and the mixture is well stirred;
4) The mixture is left for 10 days in an uncovered container at room temperature until it becomes completely fermented;

### Preferred embodiment of composition No2 and method of getting it

A Fermented product containing 68.07 units Bee honey, 1.52 units Ammonium chloride, 16.94 units Pectin, 12.71 units Vanillin and 0.76 units Vitamin E.

The preferred embodiment 2 is received in the following way:
1) 1.52 units Ammonium chloride, 16.94 units Pectin and 12.71 units Vanillin are mixed and well stirred;
2) 68.07 units Bee honey are added and the mixture is stirred until it becomes homogeneous;
3) 0.76 units Vitamin E is added and the mixture is well stirred;
4) The mixture is left for 10 days in an uncovered container at room temperature until it becomes completely fermented;

### Preferred embodiment of composition No3 and method of getting it

A Fermented product containing 77.56 units Bee honey, 2.20 units Ammonium chloride, 10.42 units Pectin, 9.40 units Vanillin and 0.42 units Vitamin E.

The preferred embodiment 3 is received in the following way:
1) 2.20 units Ammonium chloride, 10.42 units Pectin and 9.40 units Vanillin are mixed and well stirred;
2) 77.56 units bee honey are added and the mixture is stirred until it becomes homogeneous;
3) 0.42 units Vitamin E is added and the mixture is well stirred;
4) The mixture is left for 10 days in an uncovered container at room temperature until it becomes completely fermented;

### Preferred embodiment of composition No4 and method of getting it

A Fermented product containing 82.47 units Bee honey, 0.42 units Ammonium chloride, 12.71 units Pectin, 2.71 units Vanillin and 1.69 units Vitamin E.

The preferred embodiment 4 is received in the following way:
1) 0.42 units Ammonium chloride, 12.71 units Pectin and 2.71 units Vanillin are mixed and well stirred;
2) 82.47 units Bee honey are added and the mixture is stirred until it becomes homogeneous;
3) 1.69 units Vitamin E is added and the mixture is well stirred;
4) The mixture is left for 10 days in an uncovered container at room temperature until it becomes completely fermented;

### Preferred embodiment of composition No5 and method of getting it

A Fermented product containing 85.54 units Bee honey, 1.95 units Ammonium chloride, 4.54 units Pectin, 7.13 units Vanillin and 0.84 units Vitamin E.

The preferred embodiment 5 is received in the following way:
1) 1.95 units Ammonium chloride, 4.54 units Pectin and 7.13 units Vanillin are mixed and well stirred;
2) 85.54 units Bee honey are added and the mixture is stirred until it becomes homogeneous;
3) 0.84 units Vitamin E is added and the mixture is well stirred;
4) The mixture is left for 10 days in an uncovered container at room temperature until it becomes completely fermented;

### Preferred embodiment of composition No6 and method of getting it

A Fermented product containing 92.74 units Bee honey, 0.84 units Ammonium chloride, 1.86 units Pectin, 3.72 units Vanillin and 0.84 units Vitamin E.

The preferred embodiment 6 is received in the following way:
1) 0.84 units Ammonium chloride, 1.86 units Pectin and 3.72 units Vanillin are mixed and well stirred;
2) 92.74 units Bee honey are added and the mixture is stirred until it becomes homogeneous;
3) 0.84 units Vitamin E is added and the mixture is well stirred;
4) The mixture is left for 10 days in an uncovered container at room temperature until it becomes completely fermented;

### OBSERVATIONS

In order to prove the curing effect some patients suffering from insulin-dependent and non-insulin-dependent diabetes and treated with the Remedy, according to the invention, were kept under observation. The results are as follows:
i. A person suffering from non-insulin-dependent diabetes, on a diet, is, according to clinic and laboratory data, fully recovered after two courses of treatment with the Remedy (10 days, 100 g a day with preferred embodiment of composition No1).
ii. A person suffering from non-insulin-dependent diabetes, on a per-oral therapy, according to clinic and laboratory data, is fully recovered after two courses of treatment with the Remedy (10 days, 100 g a day with preferred embodiment of composition No1).
iii. A person suffering from non-insulin-dependent diabetes, on a diet, after two courses of treatment with the Remedy (10 days, 100 g a day with preferred embodiment of composition No1) still feels weak, but does not get tired easily and his blood sugar is normal.
iv. A person suffering from insulin-dependent diabetes after two courses of treatment with the Remedy (10 days, 100 g a day with preferred embodiment of composition No1) passed a year ago, is now lively and has stable blood sugar. The daily insulin dose has been reduced with 10 units (from 46 to 36 units).
v. A person suffering from insulin-dependent diabetes (46 units of insulin a day) after a course of treatment with the Remedy (20 days, 25 g a day with preferred embodiment of composition No1) feels more lively but still slightly sleepy until lunch time. He already has a normal blood sugar level by taking 36 units of insulin a day.
vi. A person suffering from insulin-dependent diabetes (56 units of insulin a day) after a course of treatment with the Remedy (20 days, 25 g a day with preferred embodiment of composition No1) feels more lively and keeps a normal blood sugar level by taking 44 units of insulin a day.

## Claims

1. Remedy for use in the prophylaxis or treatment of diabetes containing Bee honey, **wherein** it is a fermented product of Bee honey (from 68.07 to 92.74 units), Ammonium chloride (from 0.42 to 2.20 units), Pectin (from 1.86 to 16.94 units), Vanillin (from 2.71 to 12.71 units) and Vitamin E (from 0.42 to 1.69 units).

2. Remedy for use according to Claim 1, **wherein** it is a fermented product of 84.77 units of Bee honey, 1.69 units of Ammonium chloride, 4.23 units of Pectin, 8.47 units of Vanillin and 0.84 units of Vitamin E.

3. Method of getting a Remedy for use in the prophylaxis or treatment of diabetes, according to Claim 1, **wherein** certain quantities of Ammonium chloride, Pectin and Vanillin are mixed and well stirred, then Bee honey is added to the mixture and it is stirred again until becomes homogeneous, then Vitamin E is added and the mixture is stirred again and left at room temperature to ferment and the quantity of the ingredients is as follows: Ammonium chloride from 0.42 to 2.20 units, Pectin from 1.86 to 16.94 units, Vanillin from 2.71 to 12.71 units, Bee honey from 68.07 to 92.74 units and Vitamin E from 0.42 to 1.69 units.

## Patentansprüche

1. Es handelt sich hierbei um ein Mittel zur Prophylaxe und Behandlung von Diabetes, welches grundsätzlich Bienenhonig beinhaltet. Es ist ein Fermentationsprodukt von Bienenhonig (Anteil zwischen 68,07 und 92,74%), Ammoniumchlorid (Anteil zwischen 0,42 und 2,20 %), Pektin (Anteil zwischen 1,86 und 16,94 %), Vanillin (Anteil zwischen 2,71 und 12,71 %) und Vitamin E (Anteil zwischen 0,42 und 1,69 %).

2. Der Patentanspruch bezieht sich auf das Mittel nach Absatz 1, charakterisiert durch das Fermentationsprodukt von Bienenhonig (Anteil 84,77 %), Ammonium-chlorid (Anteil 1,69 %), Pektin (Anteil 4,23 %), Vanillin (Anteil 8,4 %) und Vitamin E (Anteil 0,84%).

3. Verfahren zur Herstellung dieses Mittels zur Anwendung in der Prophylaxe und Behandlung von Diabetes nach Absatz 1, Charakteristisch für dieses Mittel ist, dass bestimmte, bekannte Mengen von Ammoniumchlorid, Pektin und Vanillin gut vermischt und verrührt werden, danach wird Bienenhonig dazu gegeben und gerührt, bis eine gleichmäßige, homogene Masse entsteht. Zusätzlich wird am Ende das Vitamin E zugegeben. Nach der Vermischung wird die eingerührte, homogene Masse 10 Tage bei Zimmertemperatur ruhen gelassen, bis der Fermentationsprozess erfolgt ist, dadurch entstehen die einzelnen Bestandteile wie folgt: Ammoniumchlorid (Anteil zwischen 0,42 - 2,20 %), Pektin (Anteil zwischen 1,86 - 16,94 %), Vanillin (Anteil zwischen 2,71 - 12,71 %), Bienenhonig (Anteil zwischen 68,07 - 92,74 %), Vitamin E (Anteil zwischen 0,42 - 1,69 %).

## Revendications

1. Agent utile dans la prophylaxie ou le traitement du diabète, comprenant du miel, **caractérisée en ce qu'**un produit de la fermentation de miel de 68,07 à 92,74 parties, de chlorure d'ammonium 0,42 à 2,20 parties, de pectine de 1,86 à 16,94 parties, de vanilline 2,71 à 12,71 parties et la vitamine E de 0,42 à 1,69 parties.

2. Agent utile selon la revendication 1, **caractérisé en ce qu'**un produit de la fermentation de 84,77 parties de miel, 1,69 parties de chlorure d'ammonium, 4,23 parties de pectine, 8,47 partie de vanille et de 0,84 parties de vitamine E.

3. Procédé pour la préparation d'un agent pour utilisation dans la prophylaxie ou le traitement du diabète selon la revendication 1, **caractérisé en ce que** des certaines quantités de chlorure d'ammonium, de pectine et de vanilline sont bien mélangés et agités, puis au mélange, on ajoute le miel et agite pour obtenir un mélange uniforme, auquel est ajoutée la vitamine E et, après l'agitation, on laisse le mélange reposer 10 jours à température ambiante jusqu'à ce qu'il fermente et la quantité de constituants individuels soit la suivante: chlorure d'ammonium 0,42 à 2,20 parties, pectine de 1,86 à 16,94 parties, vanilline - 2,71 à 12,71 parties, miel est de 68,07 à 92,74 parties, la vitamine E est de 0,42 à 1,69 parties.
